# EUROPEAN PATENT APPLICATION

(11) **EP 4 850 759 A2**
(43) Date of publication of application: **09.12.2026**
(21) Application number: 26214367.0
(22) Date of filing: 26.09.2019
(51) Int. Cl.: A61P 25/18

(54) **BALIPODECT FOR TREATING OR PREVENTING AUTISM SPECTRUM DISORDERS**

(30) Priority: 28.09.2018 US 201862737985 P
(62) Document number(s) of the earlier application(s) in accordance with Art. 76 EPC: 19780031.1 / 3 856 185
(71) Applicant: Axsome Therapeutics, Inc., New York, NY 10007 (US)
(72) Inventor: MAKHIJA, Mahindra, London, WC2B 4AE (GB)
(74) Representative: Carpmaels & Ransford LLP

(57) **Abstract**

The invention is the use of a PDE10A inhibitor to treat or prevent autism spectrum disorders. More particularly, a method of treating or preventing an autism spectrum disorder selected from the group consisting of autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder, comprising administering an effective amount of 1-[2-fluoro-4-(1H-pyrazol-1-yl)phenyl]-5-methoxy-3-(1-phenyl-1H-pyrazol-5-yl)pyridazin-4(1H)-one or a salt thereof to a mammal. Additionally, a medicament for the treatment or prevention of autism spectrum disorders, and the use of a PDE10A inhibitor in the manufacture of a medicament for the treatment or prevention of autism spectrum disorders.

## Description

### Background of the Invention

Autism spectrum disorders (ASDs) are developmental disorders that affect communication and behavior. Although autism can be diagnosed at any age, it is said to be a "developmental disorder" because symptoms generally appear in the first two years of life. ASDs share a gross phenotype in terms of autism. People with ASDs have difficulty with social communication and interaction, speech, restricted interests, and repetitive behaviors, and often have behavioral issues, such as hyperactivity, and/or seizures. ASDs include, for example, autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder.

CDKL5 deficiency disorder, in particular, is a rare neuro-developmental disease resulting from the loss of function mutations in the CDKL5 gene (Xp22.13). CDKL5 is an abbreviation of "cyclin-dependent kinase-like 5". The CDKL5 gene provides instructions for making a protein that is essential for normal brain development. CDKL5 deficiency disorder, also called "Early Infantile Epileptic Encephalopathy 2", can result in an early onset of seizures (at less than 5 months old) and is similar to Rett syndrome. CDKL5 deficiency disorder can also cause intellectual disability with absent speech, sleep disturbances, hand stereotypies, slowed head growth, poor motor control, and severe mental retardation. The disorder is X-linked dominant and has higher prevalence in females, because the CDKL5 gene is located on the X chromosome (females have two X chromosomes and males have one X chromosome and one Y chromosome). There are about 1600 documented cases, but these numbers are expected to increase due to genetic screening. Currently, there is a need for a disease-modifying therapy for all patients, therapy from non-seizure symptoms, and treatment for refractory seizures.

Fragile X syndrome ("FXS"), is a genetic condition resulting from a mutation on the FMR1 (fragile X mental retardation 1) gene on the X chromosome. FMR1 codes for the protein FMRP (fragile X mental retardation protein), which is commonly found in the brain and is essential for cognitive development. Trinucleotide repeat expansion in the promoter region of FMR1 causes transcriptional silencing of FMRP production. Extensive repeat expansions in FMR1 causes a constricted appearance of the chromosome due to hypermethylation at this site. FMRP is thought to negatively regulate the translation of proteins important for development and function of excitatory synapses. FMRP is estimated to regulate the translation of about 4% of brain mRNAs. There is an overlap of phenotype for FXS, FXTAS (Fragile X Tremor Ataxia Syndrome) and FXPOI (Fragile X-associated Primary Ovarian Insufficiency). See https://www.nimh.nih.gov/labs-at-nimh/research-areas/clinics-and-labs/snpm/fragile-x-syndrome.shtml; Jonathan Ting et al., Nat. Med., 2011, 17, 1352; and Reymundo Lozano et al., Intractable Rare Dis Res. 2014, 3, 134.

FXS causes intellectual disability, behavioral and learning challenges and various physical characteristics. It is more common and more severe in males, but it also occurs in females. FXS is associated with anxiety and hyperactive behavior, such as ADHD, and seizures occur in 15% of males and 5% of females. Speech and language defects are evident by 2 years of age. Physical characteristics include narrow face and flexible fingers in 50% of patients. FXS is diagnosed by laboratory and genetic testing. Premature babies are at a higher risk. There is currently no cure to treat FXS. Some children benefit from medications that treat ADD, ADHD and other attention disorders. Other children who experience general anxiety, social anxiety, OCD and other perseverative disorders may benefit from different types of anti-anxiety medications. Other treatments include behavioral therapy. See National Fragile X Foundation, https://fragilex.org/.

Phosphodiesterases (PDEs) are a superfamily of enzymes encoded by 21 genes and subdivided into 11 distinct families according to structural and functional properties. These enzymes metabolically inactivate the ubiquitous intracellular second messengers, cyclic adenosine monophosphate (cAMP) and cyclic guanosine monophosphate (cGMP); PDEs selectively catalyze the hydrolysis of the 3'-ester bond, forming the inactive 5'-monophosphate. On the basis of substrate specificity, the PDE families can be further classified into three groups: i) the cAMP-PDEs (PDE4, PDE7, PDE8), ii) the cGMP-PDEs (PDE5, PDE6 and PDE9), and iii) the dual-substrate PDEs (PDE1, PDE2, PDE3, PDE10 and PDE11).

The cAMP and cGMP are involved in the regulation of virtually every physiological process such as pro-inflammatory mediator production and action, ion channel function, muscle relaxation, learning and memory formation, differentiation, apoptosis, lipogenesis, glycogenolysis and gluconeogenesis. Especially, in neurons, these second messengers have important role in the regulation of synaptic transmission as well as in neuronal differentiation and survival (Nat. Rev. Drug Discov. 2006, vol. 5: 660-670). Regulation of these processes by cAMP and cGMP are accompanied by activation of protein kinase A (PKA) and protein kinase G (PKG), which in turn phosphorylate a variety of substrates, including transcription factors, ion channels and receptors that regulate a variety of physiological processes. Intracellular cAMP and cGMP concentrations seem to be temporally, spatially, and functionally compartmentalized by regulation of adenyl and guanyl cyclases in response to extracellular signaling and their degradation by PDEs. See Circ. Res. 2007, vol. 100(7): 950-9667. PDEs provide the only means of degrading the cyclic nucleotides cAMP and cGMP in cells, thus PDEs play an essential role in cyclic nucleotide signaling. Thereby, PDEs could be promising targets for various therapeutic drugs.

Phosphodiesterase 10A (PDE10A) was discovered in 1999 by three independent groups (Proc. Natl. Acad. Sci. USA 1999, vol. 96: 8991-8996, J. Biol. Chem. 1999, vol. 274: 18438-18445, Gene 1999, vol. 234: 109-117). Expression studies have shown that PDE10A has the most restricted distribution within the all known PDE families; the PDE10A mRNA is highly expressed only in brain and testes (Eur. J. Biochem. 1999, vol. 266: 1118-1127, J. Biol. Chem. 1999, vol. 274: 18438-18445). In the brain, mRNA and protein of PDE10A are highly enriched in medium spiny neurons (MSNs) of the striatum (Eur. J. Biochem. 1999, vol. 266: 1118-1127, Brain Res. 2003, vol. 985: 113-126). MSNs are classified into two groups: the MSN that express D₁ dopamine receptors responsible for a direct (striatonigral) pathway and the MSN that express D₂ dopamine receptors responsible for an indirect (striatopallidal) pathway. The function of direct pathway is to plan and execution, while indirect pathway is to act as a brake on behavioral activation. As PDE10A expresses in both MSNs, PDE10A inhibitors could activate both of these pathways. The antipsychotic efficacy of current medications, D₂ or D₂/5-HT_{2A} antagonists, mainly derives from their activation of the indirect pathway in the striatum. As PDE10A inhibitors are able to activate this pathway, this suggests that PDE10A inhibitors are promising as antipsychotic drugs. The excessive D₂ receptor antagonism in the brain by D₂ antagonists causes problems of extrapyramidal side effects and hyperprolactinaemia. However, the expression of PDE10A is limited to these striatal pathways in the brain, thus side effects by PDE10A inhibitors were expected to be weaker compared with current D₂ antagonists. Regarding hyperprolactinaemia, PDE10A inhibitors would produce no prolactin elevation due to lack of D₂ receptor antagonism in the pituitary. Moreover, the presence of PDE10A in a direct pathway makes it likely that PDE10A inhibition will have some advantage over current D₂ antagonists; the direct pathway is thought to promote desired action, and activation of this pathway by PDE10A inhibitors may counteract extrapyramidal symptoms induced by excessive D₂ receptor antagonism. In addition, activation of this pathway could facilitate striatal-thalamic outflow, promoting the execution of procedural strategies. Furthermore, enhancement of second messenger levels without blockade of dopamine and/or other neurotransmitter receptors may also provide therapeutic advantages with fewer adverse side-effects compared with current antipsychotics (e.g., hyperprolactinaemia and weight gain). This unique distribution and function in the brain indicates that PDE10A represents an important new target for the treatment of neurological disorders.

PDE10A inhibitors have been reported in, for example, WO 2006/072828, WO 2008/001182, WO 2007/137819, WO 2007/137820, WO 2009/068246, WO 2009/068320, WO 2009/070583, WO 2009/070584, WO 2007/085954, WO 2007/022280, WO 2007/096743, WO 2007/103370, WO 2008/020302, WO 2008/006372, WO 2009/036766, WO 2006/028957, WO 2007/098169, WO 2007/098214, WO 2007/103554, WO 2009/025823, WO 2009/025839, WO 2007/100880, WO 2008/004117, WO 2007/082546, U.S. Patent No. 9,994,590, U.S. Patent No. 9,938,269 and US 2007/0155779.

In particular, PDE10A inhibitors are disclosed in WO 2010/090737, which is incorporated herein in its entirety. More particularly, WO 2010/090737 discloses 1-[2-fluoro-4-(1H-pyrazol-1-yl)phenyl]-5-methoxy-3-(1-phenyl-1H-pyrazol-5-yl)pyridazin-4(1H)-one (hereinafter, "Compound A") and salts thereof.

### Brief Summary of the Invention

The invention is the use of a PDE10A inhibitor to treat or prevent autism spectrum disorders. More particularly, a method of treating or preventing an autism spectrum disorder selected from the group consisting of autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder, comprising administering an effective amount of a PDE10A inhibitor to a mammal.

More particularly, the method of treating or preventing the autism spectrum disorder, wherein the PDE10A inhibitor is 1-[2-fluoro-4-(1H-pyrazol-1-yl)phenyl]-5-methoxy-3-(1-phenyl-1H-pyrazol-5-yl)pyridazin-4(1H)-one, or a salt thereof (Compound A), or a salt thereof.

More particularly, the autism spectrum disorder is CDKL5 deficiency disorder or Fragile X syndrome.

The method further comprises administering a second active ingredient with the PDE10A inhibitor to treat or prevent the autism spectrum disorder.

Accordingly, the present invention provides the following.
1. A method of treating or preventing an autism spectrum disorder selected from the group consisting of autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder, comprising administering an effective amount of a PDE10A inhibitor to a mammal.
2. The method according to 1, wherein the PDE10A inhibitor is 1-[2-fluoro-4-(1H-pyrazol-1-yl)phenyl]-5-methoxy-3-(1-phenyl-1H-pyrazol-5-yl)pyridazin-4(1H)-one, or a salt thereof.
3. The method according to 1, wherein the autism spectrum disorder is CDKL5 deficiency disorder.
4. The method according to 1, wherein the autism spectrum disorder is Fragile X Syndrome.
5. The method according to 1, further comprising administering a second active ingredient with the PDE10A inhibitor.
6. A method of treating or preventing CDKL5 deficiency disorder comprising administering an effective amount of 1-[2-fluoro-4-(1H-pyrazol-1-yl)phenyl]-5-methoxy-3-(1-phenyl-1H-pyrazol-5-yl)pyridazin-4(1H)-one or a salt thereof to a mammal.
7. A method of treating or preventing Fragile X Syndrome comprising administering an effective amount of 1-[2-fluoro-4-(1H-pyrazol-1-yl)phenyl]-5-methoxy-3-(1-phenyl-1H-pyrazol-5-yl)pyridazin-4(1H)-one or a salt thereof to a mammal.
8. A PDE10A inhibitor for use in the treatment or prevention of an autism spectrum disorder selected from the group consisting of autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder.
9. The PDE10A inhibitor according to 8, wherein the PDE10A inhibitor is 1-[2-fluoro-4-(1H-pyrazol-1-yl)phenyl]-5-methoxy-3-(1-phenyl-1H-pyrazol-5-yl)pyridazin-4(1H)-one, or a salt thereof.
10. The PDE10A inhibitor according to 8, wherein the autism spectrum disorder is CDKL5 deficiency disorder.
11. The PDE10A inhibitor according to 8, wherein the autism spectrum disorder is Fragile X Syndrome.
12. The PDE10A inhibitor according to any one of 8 to 11, wherein the PDE10A inhibitor is used in combination with a second active ingredient.
13. 1-[2-Fluoro-4-(1H-pyrazol-1-yl)phenyl]-5-methoxy-3-(1-phenyl-1H-pyrazol-5-yl)pyridazin-4(1H)-one or a salt thereof for use in the treatment or prevention of CDKL5 deficiency disorder.
14. 1-[2-Fluoro-4-(1H-pyrazol-1-yl)phenyl]-5-methoxy-3-(1-phenyl-1H-pyrazol-5-yl)pyridazin-4(1H)-one or a salt thereof for use in the treatment or prevention of Fragile X Syndrome.
15. Use of a PDE10A inhibitor in the manufacture of a medicament for the treatment or prevention of an autism spectrum disorder selected from the group consisting of autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder.
16. The use according to 15, wherein the PDE10A inhibitor is 1-[2-fluoro-4-(1H-pyrazol-1-yl)phenyl]-5-methoxy-3-(1-phenyl-1H-pyrazol-5-yl)pyridazin-4(1H)-one, or a salt thereof.
17. The use according to 15, wherein the autism spectrum disorder is CDKL5 deficiency disorder.
18. The use according to 15, wherein the autism spectrum disorder is Fragile X Syndrome.
19. The use according to any one of 15 to 18, wherein the medicament further comprises a second active ingredient.
20. Use of 1-[2-fluoro-4-(1H-pyrazol-1-yl)phenyl]-5-methoxy-3-(1-phenyl-1H-pyrazol-5-yl)pyridazin-4(1H)-one or a salt thereof in the manufacture of a medicament for the treatment or prevention of CDKL5 deficiency disorder.
21. Use of 1-[2-fluoro-4-(1H-pyrazol-1-yl)phenyl]-5-methoxy-3-(1-phenyl-1H-pyrazol-5-yl)pyridazin-4(1H)-one or a salt thereof in the manufacture of a medicament for the treatment or prevention of Fragile X Syndrome.
22. A medicament for the treatment or prevention of an autism spectrum disorder selected from the group consisting of autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder, which comprises a PDE10A inhibitor.
23. The medicament according to 22, wherein the PDE10A inhibitor is 1-[2-fluoro-4-(1H-pyrazol-1-yl)phenyl]-5-methoxy-3-(1-phenyl-1H-pyrazol-5-yl)pyridazin-4(1H)-one, or a salt thereof.
24. The medicament according to 22, wherein the autism spectrum disorder is CDKL5 deficiency disorder.
25. The medicament according to 22, wherein the autism spectrum disorder is Fragile X Syndrome.
26. The medicament according to any one of 22 to 25, wherein the medicament further comprises a second active ingredient.
27. A medicament for the treatment or prevention of CDKL5 deficiency disorder, which comprises 1-[2-fluoro-4-(1H-pyrazol-1-yl)phenyl]-5-methoxy-3-(1-phenyl-1H-pyrazol-5-yl)pyridazin-4(1H)-one or a salt thereof.
28. A medicament for the treatment or prevention of Fragile X Syndrome, which comprises 1-[2-fluoro-4-(1H-pyrazol-1-yl)phenyl]-5-methoxy-3-(1-phenyl-1H-pyrazol-5-yl)pyridazin-4(1H)-one or a salt thereof.

### Brief Description of the Drawings

Figure 1 shows the results of the Hindpaw Clasping (amplexus) test in a CDKL5 knock-out mice model.
Figures 2A and 2B show the results of an Open Field Test in a CDKL5 knock-out mice model.
Figure 3 provides a proposed mechanism of action of Compound A in CDKL5.
Figures 4A, 4B and 4C show the expression of BDNF proteins in the hippocampus, cerebellum and cortex from ELISA assays of Compound A.
Figure 5 provides the effects of 6-Methyl-2-(phenylethynyl) pyridine HCl ("MPEP") and Compound A on latency to onset of seizures in an FMR1 mouse model of Fragile X Syndrome ("the FXS mouse model").
Figure 6 provides the effects of MPEP and Compound A on the percent of mice that had a seizure in the FXS mouse model.
Figure 7 provides the effects of Compound A on total distance traveled in an Open Field Test of an FXS mouse model.
Figure 8 provides the time course on the effects of Compound A on the distance traveled during the Open Field Test in the FXS mouse model.
Figure 9 provides the effect of Compound A on contextual fear conditioning in the FXS mouse model, in particular, the average freezing behavior during a 5-minute test period.
Figure 10 provides the effect of Compound A on contextual fear conditioning in the FXS mouse model, in particular, the time course for the freezing behavior during the 5-minute test period.
Figure 11 provides the effect of Compound A on cued fear conditioning in the FXS mouse model.

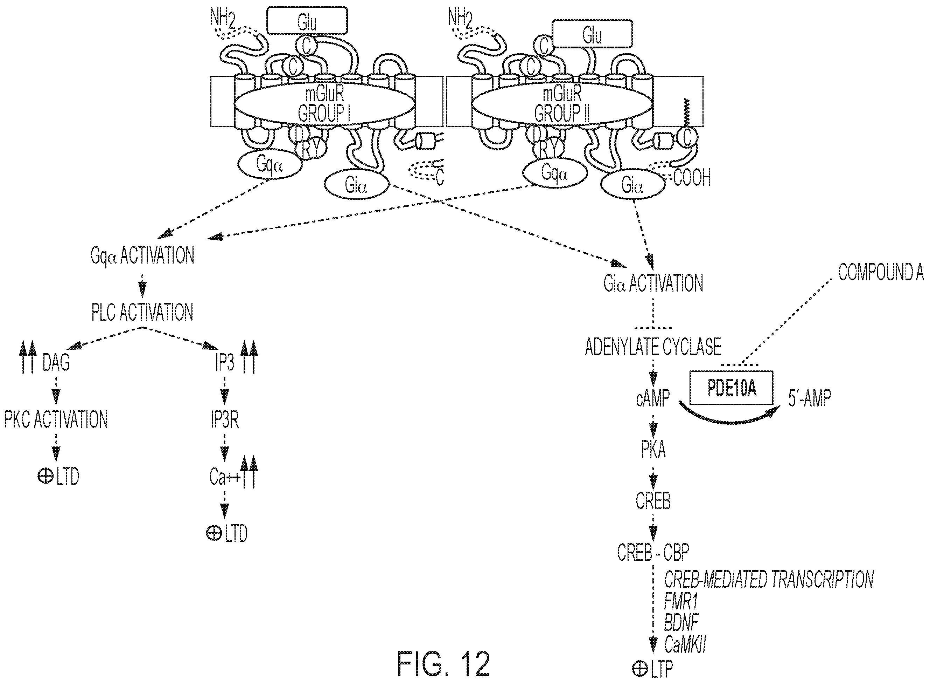

Figure 12 provides a proposed mechanism of action of Compound A in FXS adapted from Catherine Choi et al., J. Neurosci. 2015, 35, 396.

### Detailed Description of the Invention

The present inventor discovered that a PDE 10A inhibitor, namely compounds having PDE 10A inhibitory activity, such as Compound A, can treat or prevent autism spectrum disorders, such as CDKL5 deficiency disorder and Fragile X syndrome.

When the compound having PDE10A inhibitory activity, such as Compound A, is a salt, for example, metal salts, ammonium salts, salts with organic bases, salts with inorganic acids, salts with organic acids, salts with basic or acidic amino acids can be included. Preferable examples of metal salts, for example, include alkali metal salts such as sodium salts, potassium salts and the like; alkali earth metal salts such as calcium salts, magnesium salts, barium salts and the like; and aluminum salts. Preferable examples of salts with organic bases include salts with trimethylamine, triethylamine, pyridine, picoline, 2,6-lutidine, ethanolamine, diethanolamine, triethanolamine, cyclohexylamine, dicyclohexylamine, N, N'-dibenzylethylenediamine and the like. Preferable examples of salts with inorganic acids include salts with hydrochloric acid, hydrobromic acid, nitric acid, sulfuric acid, phosphoric acid and the like. Preferable examples of salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of salts with basic amino acids include salts with arginine, lysine, ornithine and the like. Preferable examples of salts with acidic amino acids include salts with aspartic acid, glutamic acid and the like. Among them, salts that are pharmacologically acceptable are preferable. For example, in the case when acidic functional group are present in the compound, for example, inorganic salts including alkali metal salts (e.g., sodium salts, etc.) and alkali earth metal salts (e.g., calcium salts, magnesium salts, barium salts, etc.) and ammonium salts are preferable. In contrast, in the case when basic functional group are present in the compound, for example, salts with inorganic acids such as hydrochloric acid, hydrobromic acid, nitric acid, sulfuric acid, phosphoric acid, etc. or salts with organic acid such as acetic acid, phthalic acid, fumaric acid, oxalic acid, tartaric acid, maleic acid, citric acid, succinic acid, methanesulfonic acid, p-toluenesulfonic acid, etc. are preferable.

The compounds having PDE10A inhibitory activity, such as Compound A, are safe and useful in a method treating and preventing the autism spectrum diseases and symptoms thereof in mammals, such as humans, cows, horses, dogs, cats, monkeys, mice, rats, etc., and particularly in humans.

The compounds having PDE10A inhibitory activity, such as Compound A, can be administered in a dosage form which is manufactured according to a per se known method for manufacturing pharmaceutical formulations (e.g., methods described in Japanese Pharmacopoeia) such as tablets (inclusive of sugar coated tablet, film coated tablet, sublingual tablet, orally disintegrable tablet, and buccal), pills, powders, granules, capsules (inclusive of soft capsule, and microcapsule), troches, syrups, liquid dosage forms, emulsions, controlled-release preparations(e.g., quick-release preparation, sustained-release preparation, sustained-release microcapsule ), aerosols, films (e.g., orally disintegrable film, adhesive film for application to oral- cavity mucosa), injections (e.g., subcutaneous injection, intravenous injection, intramuscular injection, intraperitoneal injection), drip infusion, percutaneous absorbent, ointment, lotion, patch, suppositories (e.g., rectal suppository, vaginal suppository), pellets, transnasal preparations, pulmonary preparations (inhalant), eye drops and the like, in an oral or parenteral route (e.g., intravenous, intramuscular, subcutaneous, intraorgan, intranasal, intradermal, ophthalmic instillation, intracerebral, intrarectal, intravaginal, intraperitoneal, directly to lesion).

The pharmaceutical formulation (also referred to as a pharmaceutical composition or as a medicament) may contain a pharmaceutical acceptable carrier. As a pharmaceutical acceptable carrier of the compounds having PDE10A inhibitory activity, such as Compound A, common organic or inorganic carrier substances are used as formulation raw materials. Carriers are added as vehicles, lubricants, binders and disintegrants in the solid formulations; and as solubilizing agents, suspending agents, isotonization agents, buffers and soothing agents in the liquid formulations. If desired, formulation additives such as antiseptics, antioxidants, colorants, sweeteners, etc. can be used.

The content of the compounds having PDE10A inhibitory activity, such as Compound A, in the method of treating or preventing the autism spectrum disorder, such as CDKL5 deficiency disorder or Fragile X syndrome, in the pharmaceutical compositions varies based on the dosage forms, dosages of the compound of the present invention, etc. For example, the content approximately ranges from 0.01 to 100 wt% and preferably from 0.1 to 95 wt% relative to the entire amount of the composition.

The dosage depends upon injection targets, administration routes, target diseases, symptoms, etc. For example, in the case of oral administration in patients with CDKL5 deficiency disorder or Fragile X Syndrome (adults, bodyweight of approximately 60 kg), generally a single dose ranges from approximately 0.1 to 30 mg/kg bodyweight, preferably from approximately 0.2 to 10 mg/kg bodyweight, further preferably from approximately 0.5 to 10 mg/kg bodyweight, and this dosage is preferably administered once daily or several times daily (e.g., 3 times).

The compounds can be administered as the sole active agent or in combination with other pharmaceutical agents (also referred to as a second active ingredient), such as other agents used in the treatment or prevention of an autism spectrum disorder. In such combinations, each active ingredient can be administered either in accordance with their usual dosage range or a dose below their usual dosage range, and can be administered either simultaneously or sequentially.

### Examples

The present invention will be explained in detail below with reference to Examples. Because these are simply examples, the present invention will not be limited to these examples and the present invention can be modified in the range not deviating from the scope of the present invention.

### Example 1: Hindpaw Clasping (amplexus)

A hind paw clasping test was performed in accordance with the procedures outlined by Wang et al., PNAS, vol. 109, no. 52, pp. 21516-21521 (2012). The test is done in a CDKL5 knock-out mice model. Mice are suspended for a two-minute trial. If clasping occurs for two seconds, the mouse is positive for CDKL5 deficiency, which is a neurological impairment. Wang et al. noted the CDKL5 mutant mice, but no quantification was made. Tang et al., J. Neurosci. 37(31):7420-7437 (2017) reported 17/18 (94%) hemi male mice were positive for the clasp phenotype.

The following groups of mice were compared:
- Group (1) C57BL/6 mice with a vehicle
- Group (2) CDKL5 male hemizygous (-/Y) mice with a vehicle
- Group (3) CDKL5 male hemizygous (-/Y) mice with a PDE10A inhibitor (Compound A)

Mice were treated with an inhibitor compound (Compound A, 5 mg/kg once per day) or a vehicle by oral gavage for seven days prior to assay initiation, and then throughout the duration of the assays. Neurobehavioral assays were run when the mice were 8-10 weeks old. Tissues were collected at the end of the study to determine plasma and CNS PK.

Group (3) CDKL5/-Y mice were treated with Compound A, which is a PDE10A inhibitor.

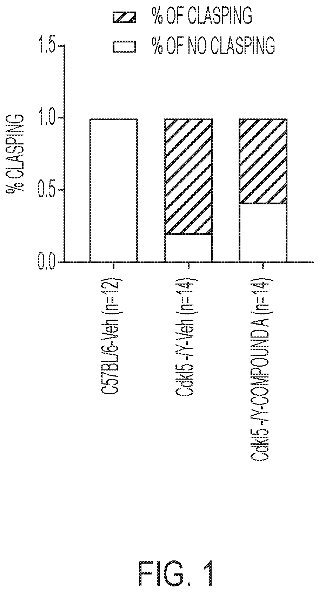

The results are shown in Fig. 1. If clasping occurs for two seconds, the mouse is positive for CDKL5 deficiency. Group (3) increased the percentage of no clasping compared with the Group (2) CDKL5/-Y mice given only the vehicle.

### Example 2: Open Field Test

Spontaneous activity in an Open Field Arena is commonly used to assess general activity and ambulation. Mice were placed in the center of an arena for a fifteen-minute trial. The horizontal, vertical (rearing) and center activity are dependent variables. CDKL5/-Y mice show increased horizontal activity in the open field, suggesting impairment of general motor function (hyperactivity). Mice corresponding to the Groups (1) to (3) in Example 1 were compared, and were given Compound A or a vehicle the same way as in Example 1.

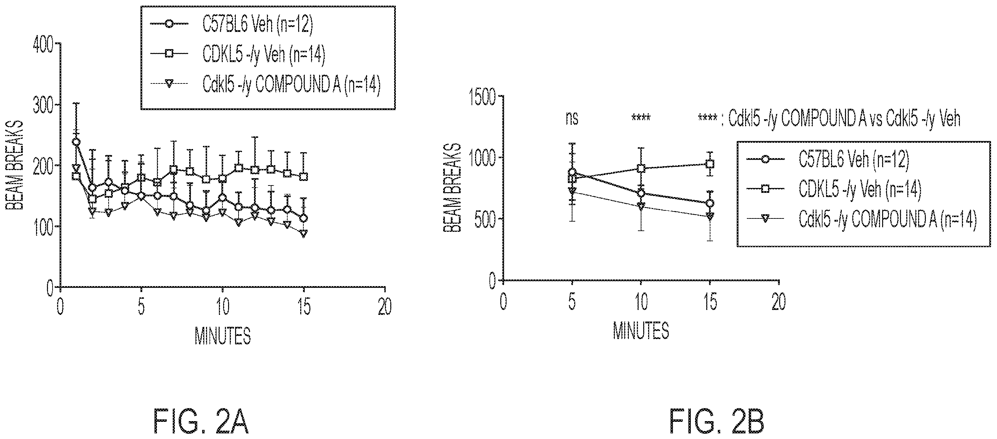

The results of the Open Field Test are shown in Figs. 2A and 2B. Fig. 2A provides the results per minute from 0 to 15 minutes. Fig. 2B provides the results in five-minute intervals from 5 to 15 minutes. The Group (3) mice administered with Compound A significantly suppressed the total distance traveled in the open field in the CDKL5-/Y mice compared to Groups (1) and (2). Accordingly, Group (3) showed a decrease in hyperactivity.

The results of Examples 1 and 2 (the hindpaw clasping and open field tests) indicate that Compound A suppresses the induction of motor deficits in the CDKL5 mouse model.

### Example 3: Plasma and Brain Exposure Analysis

### An in vitro profile of the PDE10A inhibitor was created for the CDKL5-/Y mice.

| | |
|---|---|
| PDE 10A Inhibitor | Compound A: 1-[2-fluoro-4-(1H-pyrazol-1-yl)phenyl]-5-methoxy-3-(1-phenyl-1H-pyrazol-5-yl)pyridazin-4(1H)-one |
| Molecular Weight | 428.42 |
| *in vitro* assay IC₅₀ | Human PDE10A2 0.30 nmol/L |
| fu,plasma | 0.116 |
| IC₅₀ *in vivo** | 1.1 ng/mL |

$* {IC}_{50} in vivo \left(ng/mL\right) = {IC}_{50} \left(nM\right) \times M . W . \times 1000 / fu , plasma$

### IC₅₀ in vivo (1.1 ng/mL) is a target plasma concentration for Compound A

The following Table 1 shows the concentrations of Compound A in the mouse plasma and mouse brain after Compound A administration under the following procedures:
Animals: CDKL5-/Y mice
Route: oral administration
Dose: 5 mg/kg
Regimen: once per day
Duration: 14 days
Time point: 24 hours after the last dose
N= 6-7

**Table 1: Concentrations in the mouse plasma and mouse brain after Compound A administration**

| | Matrix | Plasma | Brain |
|---|---|---|---|
| | Analyte | Compound A | Compound A |
| Sample No. | LLOQ | 0.1 (ng/mL) | 0.5 (ng/g) |
| 1 | | 7.0 | |
| 2 | | 3.1 | |
| 3 | | 4.3 | |
| 4 | | 8.0 | |
| 5 | | 4.4 | |
| 6 | | 4.8 | |
| 7 | | 2.8 | |
| 8 | | | 36.3 |
| 9 | | | 48.2 |
| 10 | | | 38.9 |
| 11 | | | 64.5 |
| 12 | | | 44.9 |
| 13 | | | 43.8 |

| | | |
|---|---|---|
| Mean | 4.9 | 46.1 |
| Standard Deviation | 1.9 | 10.0 |

In Table 1, "LLOQ" is the "lower limit of quantification", and it is used to indicate the sensitivity of the assays. The numbers in plasma and brain columns are in ng/mL for plasma and ng/g for brain.

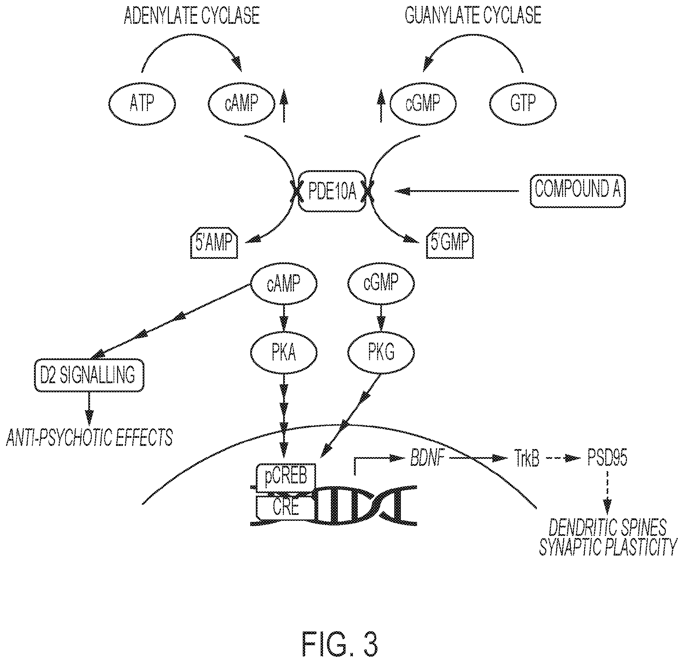

Table 1 shows that the plasma concentration in all mice was higher than the target IC₅₀ *in vivo* of Compound A (1.1 ng/mL). The plasma concentration of Compound A in the CDKL5-/Y mice covered the target plasma concentration even at 24 hours after the last administration. The brain concentration in the CDKL5-/Y mice suggested that a positive target engagement-pharmacodynamics (TE-PD) effect would be expected. Pharmacokinetic (PK) analysis in the efficacy study suggested that positive efficacy of the PDE10A inhibitor Compound A in CDKL5-Y would be reasonable and mechanism-based. Fig. 3 provides a proposed mechanism of action of Compound A in CDKL5.

In summary, the CDKL5 male hemizygous (-/Y) mice demonstrate a significantly increased overall activity or lack of habituation (as seen in the Open Field Test) and clasping phenotype at 8-10 weeks of age. The PDE10A inhibitor Compound A significantly normalized the hyperlocomotion seen in CDKL5 male hemizygous (-/Y) mice and improved the clasping behavior. PK analysis of plasma and brain samples indicate sufficient exposure of Compound A in these compartments and suggest target engagement.

### Example 4: BDNF Assay

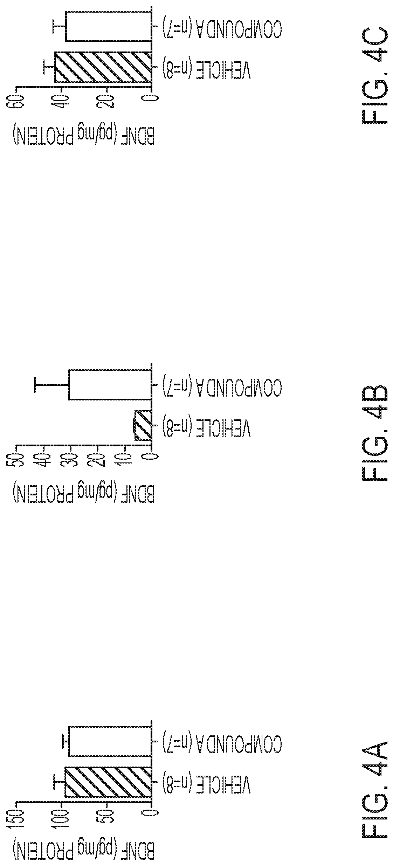

The expression of BDNF proteins in hippocampus, cerebellum and cortex were assayed by ELISA. The results are shown in Fig. 4A, Fig. 4B and Fig. 4C, respectively, in which Compound A was assayed.

### Example 5: Suppression of Audiogenic Seizures in an FMR1 Mouse Model of Fragile X Syndrome

### The tested animals

Male FMR1 knockout mice were bred at PsychoGenics. The mice were housed in OPTI-Mice ventilated cages for the duration of the study. All mice were acclimated to the environment, examined, handled, and weighed prior to initiation of the study to assure adequate health and suitability and to minimize non-specific stress associated with manipulation. During the course of the study, 12/12 light/dark cycles were maintained. The room temperature was maintained between 20 and 23°C with a relative humidity maintained around 50%. Chow and water were provided *ad libitum* for the duration of the study. Each mouse was randomly assigned across treatment groups. The testing was performed during the animals' light cycle phase at 3 weeks of age.

### Pre-Treatment

Prior to audiogenic seizures, on test day, three groups of mice were pre-treated as follows:
Group (1): a vehicle orally administered at a dose volume of 10 mL/kg, 150 minutes prior to testing.
Group (2): 6-Methyl-2-(phenylethynyl) pyridine HCl ("MPEP") (Sigma Aldrich; 30 mg/kg) was dissolved in sterile injectable saline and was administered by intraperitoneal injection at a dose volume of 10 mL/kg, 30 minutes prior to testing. (MPEP is an mGlu5 receptor antagonist.)
Group (3): Compound A (5 mg/kg) was dissolved in 0.5% methylcellulose and orally administered at a dose volume of 10 mL/kg, 150 minutes prior to testing.

### Behavioral Testing

After the pre-treatments, the mice of Groups (1)-(3) were individually placed in a Plexiglas chamber and allowed to explore for 15 seconds. Then, they were exposed to a 125 dB tone. The observer was blinded to the pre-treatment conditions during the test. The mice were scored by the observer based on their response, latency, and seizure intensity during a 5-minute test as follows:

| | |
|---|---|
| 0: | no response |
| 1: | wild running and jumping |
| 2: | clonic seizures |
| 3: | clonic-tonic seizures |
| 4: | tonic seizures |
| 5: | respiratory arrest |

The following endpoints were reported. Mice exhibiting no response were given latency scores of 300 seconds for data analysis purposes:

| | |
|---|---|
| 1. | Latency to seizure (max 300 sec. if no seizure) |
| 2. | Percent seizure |
| 3. | Seizure score |

### Results

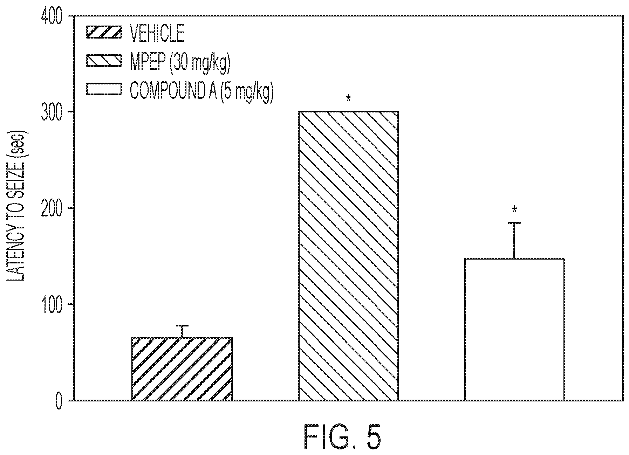

One-way analysis of variance ("one-way ANOVA") found a significant treatment effect. Post hoc analysis showed that Group (2) MPEP and Group (3) Compound A increased the latency to seizure compared to the vehicle Group (1). The effects on latency to onset of seizure are shown in Fig. 5. Data are presented as mean ± SEM (standard error to the mean). *p<0.05 indicates a significant difference compared to the vehicle treated Group (1).

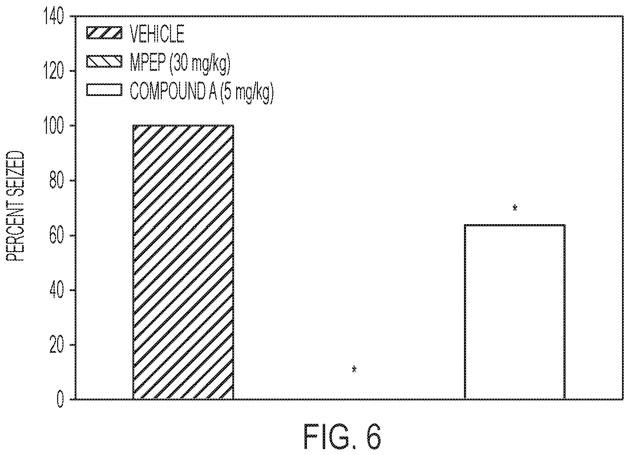

N-1 Chi-Square test found a significant treatment effect of decreasing seizures. Post hoc analysis showed that Group (2) MPEP and Group (3) Compound A significantly decreased seizure rates compared to the vehicle treated Group (1). These effects are shown in Fig. 6. Data are presented as a percentage of mice seized. *p<0.05 indicates a significant difference compared to the vehicle treated Group (1).

### Example 6: Open Field Test in an FMR1 Mouse Model of FXS

### The tested animals

Male FMR1 knockout ("KO") mice and wild-type ("WT") mice were bred at PsychoGenics. These mice were handled and chosen according to Example 5. However, the open field test started at 10 weeks of age, following 2 weeks of dosing. Three groups of mice were tested as follows:
Group (1): WT - Vehicle Group: a vehicle was orally administered for two weeks at a dose volume of 10 mL/kg. On testing days, the vehicle was administered 150 minutes prior to testing.
Group (2): FMR1 KO - Vehicle Group: a vehicle was orally administered for two weeks at a dose volume of 10 mL/kg. On testing days, the vehicle was administered 150 minutes prior to testing.
Group (3): FMR1 KO - Compound A Group: Compound A (5 mg/kg) was dissolved in 0.5% methylcellulose and orally administered for two weeks at a dose volume of 10 mL/kg. On testing days, Compound A was administered 150 minutes prior to testing.

### Test Conditions and Results

Open field chambers made of Plexiglas square chambers (27.3 x 27.3 x 20.3 cm; Med Associates Inc., St Albans, VT) surrounded by infrared photo beams (16 x 16 x 16) were used to measure horizontal and vertical activity of tested mice. The mice were brought into the chamber for at least 1 hour acclimation to the experimental room conditions prior to testing. Following 150 minutes of pre-treatment, the mice were placed in the center of the chamber for a 60-minute test period. After the 60-minutes, the mice were placed back into their home cage. During the test period, locomotor activity was measured in 5 minute intervals and total distance traveled was measured.

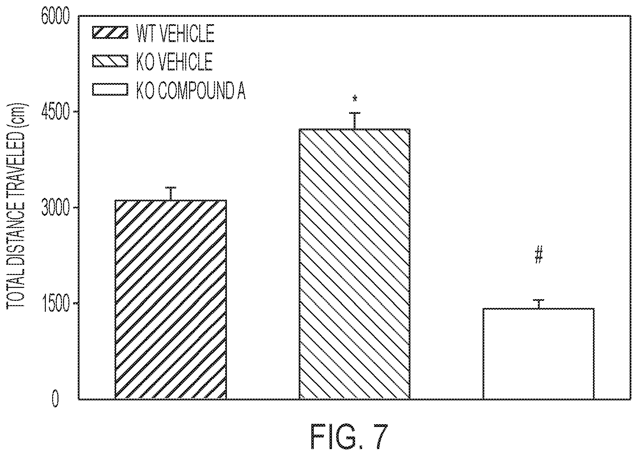

The total distance traveled in the open field during the 60-minute test period for each Group is shown in Fig. 7. Data are presented as mean ± SEM. *p<0.05 indicates a significant difference compared to WT - Vehicle Group (1). #p<0.05 indicates a significant compared to FMR1 KO - Vehicle Group (2).

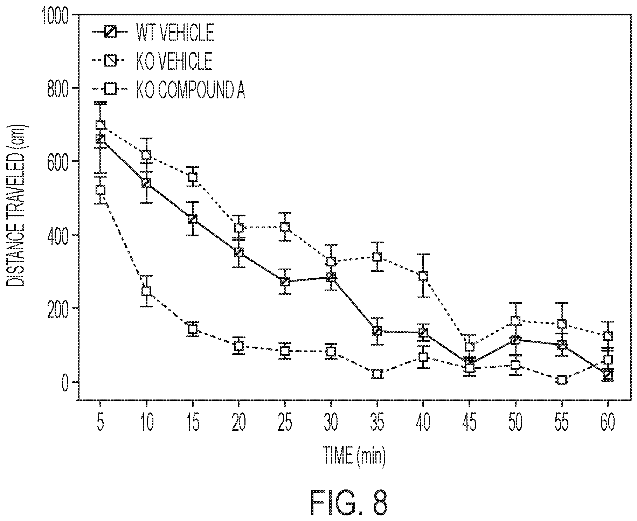

The time course on the effects of Compound A for distance traveled among Groups (1)-(3) is shown in Fig. 8. Data are presented as mean ± SEM.

The results of Figs. 7 and 8 show that Compound A suppresses hyperactivity seen in the FXS mouse model.

### Example 7: Fear Conditioning Test in an FMR1 Mouse Model of FXS

### Testing

After completion of the open field test described in Example 6, a Fear Conditioning test was conducted on the mice of Groups (1)-(3) in a fear conditioning system manufactured by Coulbourn Instruments (PA, USA).

On day 1, mice were placed in a conditioning chamber to habituate to the context for 2 minutes (CS). A tone was presented for 20 seconds. 30 seconds after the CS ended, a foot shock (1s, 0.5 mA) was presented (US). The pairing of the CS and US was repeated a total of 3 times, with an interval of 60 seconds between pairings. Mice remained in the conditioning chamber for another 60 seconds, and then returned to their home cage.

In the morning of day 2, mice were tested for contextual memory. Mice were placed in chambers for 5 minutes. In the afternoon of day 2, mice were tested for cued memory. Mice were placed in the conditioning chamber to habituate to the context for 2 minutes (Pre-Cue). Then, the CS was presented for a total of three times for 20 seconds, with 60 second inter-trial intervals. Freezing behavior, defined as the complete lack of movement, was captured automatically with a video system and FreezeView software (Coulbourn Instruments, PA,

### USA).

### Results

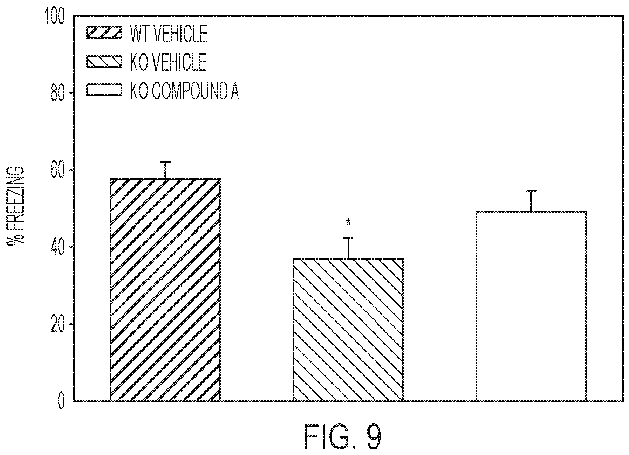

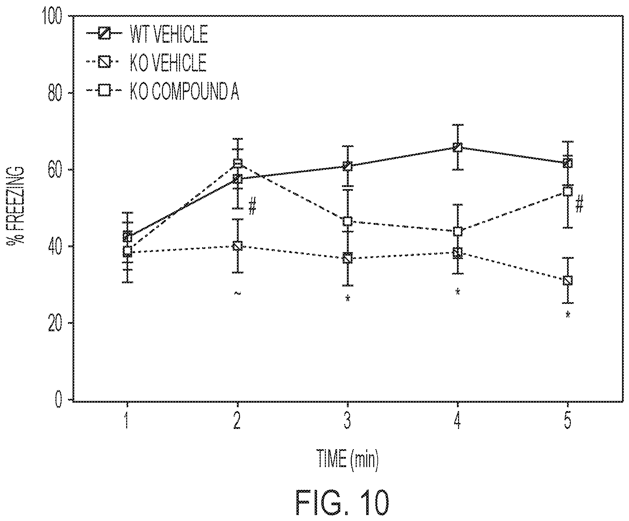

The effect of Compound A is shown in Figs. 9 and 10. The average freezing during the 5 minute test is represented in Fig. 9. Data are presented as mean SEM. *p<0.05 indicates a difference compared to the WT - Vehicle Group (1).

One way ANOVA found a significant treatment effect. FMR1 KO - Vehicle Group (2) showed a significant decrease in freezing behavior compared to the WT - Vehicle Group (1).

The time course for freezing behavior during the 5 minute test is shown in Fig. 10. Data are presented as SEM. *p<0.05 indicates a significant difference compared to the WT - Vehicle Group (1). #p<0.05 indicates a significant difference compared to the FMR1 KO - Vehicle Group (2). ~p<0.09 indicates a significant difference compared to the WT - Vehicle Group (1).

The vehicle-treated FMR1 mice (Group (2)) showed a decreased freezing response during minutes 3 to 5 of the test compared to the vehicle-treated WT mice (Group (1)). The Compound-A treated mice (Group (3)) showed an increased freezing response during minute 2 and minute 5 compared to the vehicle-treated WT mice (Group (1)).

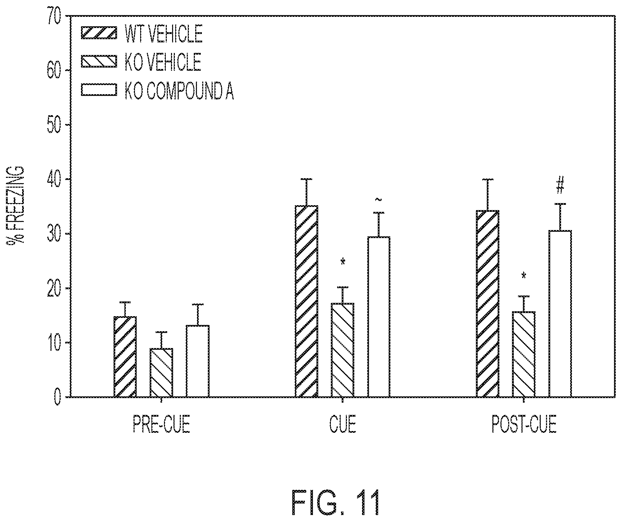

The effect of Compound A freezing behavior during the cued fear conditioning test is shown in Fig. 11. Data are presented as mean SEM. *p<0.05 indicates a significant difference compared to the WT - Vehicle Group (1). #p<0.05 indicates a significant difference compared to the FMR1 KO - Vehicle Group (2). ~p<0.09 indicates a significant difference compared to the FMR1 KO - Vehicle Group (2).

During Pre-Cue, ANOVA found no significant differences between the treatment groups. During Cue response, ANOVA found a significant treatment effect. The vehicle-treated FMR1 mice (Group (2)) showed a significant decrease in freezing behavior compared to the vehicle-treated WT mice (Group (1)). Treatment with Compound A showed a strong trend to increasing freezing response in FMR1 mice (p=0.06). Similarly, during Post-Cue response, ANOVA found a significant treatment effect. The vehicle-treated FMR1 mice (Group (2)) showed significant decrease in freezing behavior compared to the vehicle-treated WT mice. (Group (1)). The treatment with Compound A increased freezing response in the FMR1 mice.

### Plasma and Brain Collections

Plasma and brains were collected from all mice tested in open field and fear conditioning tests.

For plasma collection, mice were decapitated and trunk blood collected in K2EDTA tubes and kept on ice for short-term storage. Within 15 minutes of blood collection, tubes were centrifuged for 15 minutes at 3,000 g and in a refrigerated centrifuge. Plasma was extracted into pre-labeled tubes. Samples were stored at -80°C.

For brain collections, the following brain samples were collected:
Group (1): WT - Vehicle Group. The brain was divided into two hemispheres. One hemisphere was weighed and frozen on dry ice for BDNF analysis. The other hemisphere was discarded. Samples were stored at -80°C.
Group (2): KO - Vehicle Group. The brain was divided into two hemispheres. One hemisphere was weighed and frozen on dry ice for BDNF analysis. The other hemisphere was discarded. Samples were stored at -80°C.
Group (3): KO - Compound A Group. The brain was divided into two hemispheres. One hemisphere was weighed and frozen on dry ice for BDNF analysis. The other hemisphere was homogenized and frozen on dry ice. Samples were stored at -80°C.

In summary, Examples 5-7 show that Compound A is a highly selective PDE10A inhibitor and works by increasing cyclic nucleotide levels (cAMP and cGMP). Compound A rescues the phenotype seen in FXS mice in terms of (1) suppressing audiogenic induced seizures, (2) inhibiting hyperlocomotion in FXS mice, and (3) improving cognition in cued and context fear conditioning assays (significantly in cued-fear conditioning).

This application is based on U.S. Provisional Application No. 62/737,985 filed in United States, the contents of which are encompassed in full herein and incorporated herein by reference in their entirety.

### Numbered embodiments of the disclosure

1. A method of treating or preventing an autism spectrum disorder selected from the group consisting of autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder, comprising administering an effective amount of a PDE10A inhibitor to a mammal.
2. The method according to embodiment 1, wherein the PDE10A inhibitor is 1-[2-fluoro-4-(1H-pyrazol-1-yl)phenyl]-5-methoxy-3-(1-phenyl-1H-pyrazol-5-yl)pyridazin-4(1H)-one, or a salt thereof.
3. The method according to embodiment 1, wherein the autism spectrum disorder is CDKL5 deficiency disorder.
4. The method according to embodiment 1, wherein the autism spectrum disorder is Fragile X Syndrome.
5. The method according to embodiment 1, further comprising administering a second active ingredient with the PDE10A inhibitor.
6. A method of treating or preventing CDKL5 deficiency disorder comprising administering an effective amount of 1-[2-fluoro-4-(1H-pyrazol-1-yl)phenyl]-5-methoxy-3-(1-phenyl-1H-pyrazol-5-yl)pyridazin-4(1H)-one or a salt thereof to a mammal.
7. A method of treating or preventing Fragile X Syndrome comprising administering an effective amount of 1-[2-fluoro-4-(1H-pyrazol-1-yl)phenyl]-5-methoxy-3-(1-phenyl-1H-pyrazol-5-yl)pyridazin-4(1H)-one or a salt thereof to a mammal.
8. A PDE10A inhibitor for use in the treatment or prevention of an autism spectrum disorder selected from the group consisting of autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder.
9. The PDE10A inhibitor according to embodiment 8, wherein the PDE10A inhibitor is 1-[2-fluoro-4-(1H-pyrazol-1-yl)phenyl]-5-methoxy-3-(1-phenyl-1H-pyrazol-5-yl)pyridazin-4(1H)-one, or a salt thereof.
10. The PDE10A inhibitor according to embodiment 8, wherein the autism spectrum disorder is CDKL5 deficiency disorder.
11. The PDE10A inhibitor according to embodiment 8, wherein the autism spectrum disorder is Fragile X Syndrome.
12. The PDE10A inhibitor according to embodiment 8, wherein the PDE10A inhibitor is used in combination with a second active ingredient.
13. 1-[2-Fluoro-4-(1H-pyrazol-1-yl)phenyl]-5-methoxy-3-(1-phenyl-1H-pyrazol-5-yl)pyridazin-4(1H)-one or a salt thereof for use in the treatment or prevention of CDKL5 deficiency disorder.
14. 1-[2-Fluoro-4-(1H-pyrazol-1-yl)phenyl]-5-methoxy-3-(1-phenyl-1H-pyrazol-5-yl)pyridazin-4(1H)-one or a salt thereof for use in the treatment or prevention of Fragile X Syndrome.
15. Use of a PDE10A inhibitor in the manufacture of a medicament for the treatment or prevention of an autism spectrum disorder selected from the group consisting of autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder.
16. The use according to embodiment 15, wherein the PDE10A inhibitor is 1-[2-fluoro-4-(1H-pyrazol-1-yl)phenyl]-5-methoxy-3-(1-phenyl-1H-pyrazol-5-yl)pyridazin-4(1H)-one, or a salt thereof.
17. The use according to embodiment 15, wherein the autism spectrum disorder is CDKL5 deficiency disorder.
18. The use according to embodiment 15, wherein the autism spectrum disorder is Fragile X Syndrome.
19. The use according to embodiment 15, wherein the medicament further comprises a second active ingredient.
20. Use of 1-[2-fluoro-4-(1H-pyrazol-1-yl)phenyl]-5-methoxy-3-(1-phenyl-1H-pyrazol-5-yl)pyridazin-4(1H)-one or a salt thereof in the manufacture of a medicament for the treatment or prevention of CDKL5 deficiency disorder.
21. Use of 1-[2-fluoro-4-(1H-pyrazol-1-yl)phenyl]-5-methoxy-3-(1-phenyl-1H-pyrazol-5-yl)pyridazin-4(1H)-one or a salt thereof in the manufacture of a medicament for the treatment or prevention of Fragile X Syndrome.

## Claims

1. A PDE110A inhibitor for use in the treatment or prevention of an autism spectrum disorder selected from the group consisting of autistic disorder, CDKL5 deficiency disorder, childhood disintegrative disorder, Rett syndrome, Fragile X syndrome, Kleefstra syndrome, Pitt Hopkins syndrome, Angelman syndrome, Kabuki syndrome, Asperger's syndrome, Heller's syndrome and Pervasive Developmental Disorder.

2. The PDE10A inhibitor for use according to claim 1, wherein the PDE10A inhibitor is 1-[2-fluoro-4-(1H-pyrazol-1-yl)phenyl]-5-methoxy-3-(1-phenyl-1H-pyrazol-5-yl)pyridazin-4(1H)-one, or a salt thereof.

3. The PDE10A inhibitor for use according to claim 1 or 2, wherein the autism spectrum disorder is CDKL5 deficiency disorder.

4. The PDE10A inhibitor for use according to claim 1 or 2, wherein the autism spectrum disorder is Fragile X Syndrome.

5. The PDE10A inhibitor for use according to any preceding claim, wherein the PDE10A inhibitor is used in combination with a second active ingredient.

6. 1-[2-Fluoro-4-(1H-pyrazol-1-yl)phenyl]-5-methoxy-3-(1-phenyl-1H-pyrazol-5-yl)pyridazin-4(1H)-one or a salt thereof for use in the treatment or prevention of CDKL5 deficiency disorder.

7. 1-[2-Fluoro-4-(1H-pyrazol-1-yl)phenyl]-5-methoxy-3-(1-phenyl-1H-pyrazol-5-yl)pyridazin-4(1H)-one or a salt thereof for use in the treatment or prevention of Fragile X Syndrome.
